# EUROPEAN PATENT SPECIFICATION

(11) **EP 1 715 848 B1**
(45) Date of publication and mention of the grant of the patent: **28.07.2010**
(21) Application number: 05707213.4
(22) Date of filing: 04.02.2005
(51) Int. Cl.: A61K 9/107, A61K 31/55, A61K 31/164

(54) **MICROEMULSION FORMULATIONS COMPRISING PARTICULAR SUBSTANCE P ANTAGONISTS**
MIKROEMULSIONSFORMULIERUNGEN MIT BESTIMMTEN SUBSTANZ P ANTAGONISTEN
FORMULATIONS SOUS FORME DE MICRO-EMULSION RENFERMANT CERTAINS ANTAGONISTES DE LA SUBSTANCE P

(30) Priority: 06.02.2004 GB 0402679
(43) Date of publication of application: 02.11.2006
(73) Proprietor: Novartis AG, 4056 Basel (CH); Novartis Pharma GmbH, 1230 Wien (AT)
(72) Inventor: LÜCKEL, Barbara, 79540 Lörrach (DE); BUEB, Waltraud, 4663 Aarburg (CH); OTTINGER, Isabel, 79111 Freiburg (DE); REINHART, Thomas, 79540 Lörrach (DE); RIES,Angelika, 4224 Nenzlingen (CH)
(74) Representative: Krauss, Jan
(86) International application number: PCT/EP2005/001166
(87) International publication number: WO 2005/074891

(56) References cited:
- US-B1- 6 319 917
- GERSPACHER M ET AL: "Dual neurokinin NK1/NK2 antagonists: N-[(R,R)-(E)-1-arylmethyl-3-(2- oxo-azepan-3-yl)carbamoyl]allyl-N-methyl-3 ,5- bis(trifluoromethyl)benzamides and 3-[N'-3,5- bis(trifluoromethyl)benzoyl-N-arylmethyl-N '-methylhydrazino]-N-[(R)- 2-oxo-azepan-3-yl]propionamides" BIOORGANIC & MEDICINAL CHEMISTRY LETTERS, OXFORD, GB, vol. 11, no. 23, 2001, pages 3081-3084, XP002240091 ISSN: 0960-894X
- ZABKA M: "Colloidal drug delivery systems - Current state and perspectives III. Microemulsions as drug delivery vehicles" FARMACEUTICKY OBZOR 2003 SLOVAKIA, vol. 72, no. 4-5, 2003, pages 87-92, XP009049343 ISSN: 0014-8172
- HAUSS D J: "Lipid-based systems for oral drug delivery: Enhancing the bioavailability of poorly water soluble drugs" AMERICAN PHARMACEUTICAL REVIEW 2002 UNITED STATES, vol. 5, no. 4, 2002, pages 22-28, XP001206462 ISSN: 1099-8012

## Description

The present invention relates to novel pharmaceutical compositions in which the active agent is a substance P antagonist, in particular a 5-aryl-4(R)-arylcarbonylamino-pent-2-enoic acid amide, that is useful for the treatment and prevention of respiratory diseases including asthma and chronic obstructive pulmonary disease, bowel disorders including irritable bowel syndrome (IBS), urinary incontinence, and cough.

From Gerspacher et al. (Bioorg. Med. Chem. Lett. 2001; 11:3081-3084), two dual neurokinin NK(1)/NK(2) antagonists are known, namely N-[(R,R)-(E)-1-arylmethyl-3-(2-oxo-azcpan-3-yl)carbamoyl]allyl-N-methyl-3,5-bis(trifluoromethyl)benzamides and 3-[N'-3,5-bis(trifluoromethyl)bcnzoyl-N-arylmethyl-N'-methylhydrazino]-N-[(R)-2-oxo-azepan-3-yl]propionamides.

M. Zabka (Farmaceuticky Obzor 2003 Slovakia; 72: 97-92) describes colloidal drug delivery systems and microemulsions as drug delivery vehicles.

D. J. Hauss describes lipid-based systems for oral drug delivery and in particular the enhancement of the bioavailability of poorly water soluble drugs (American Pharmaceutical Review 2002; 5: 22-28).

5-atyl-4(R)-arylcarbonylamino-pent-2-enoic acid amide substance P antagonists are a class of compounds described in international patent application WO 98/07694.

5-aryl-4(R)-arylcarbonylamino-pent-2-enoic acid amide substance P antagonists, such as those disclosed in WO 98/07694, present highly specific difficulties in relation to administration generally and galenic compositions in particular, including in particular problems of drug bioavailability and variability in inter- and intra-patient dose response, necessitating development of a non-conventional dosage form.

In accordance with the present invention it has now surprisingly been found that stable pharmaceutical compositions with 5-aryl-4(R)-arylcarbonylamino-pent-2-enoic acid amide substance P antagonists, having particularly interesting bioavailability characteristics and reduced variability in inter- and intra-subject bioavailability parameters, arc obtainable. These novel compositions have been found to meet or substantially reduce the difficulties encountered previously. It has been shown that the compositions of the invention may enable effective dosaging with concomitant enhancement of bioavailability as well as reduced variability of absorption/bioavailability levels for and between individual patients. Thus, the invention may achieve elective therapy with tolerable dosage levels of such 5-aryl-4(R)-arylcarbonylamino-pent-2-enoie acid amide substance P antagonists, and may permit closer standardization and optimization of daily dosage requirements for each individual.

Consequently, occurrence of potential undesirable side-cffeets is diminished and overall cost of therapy may be reduced.

In a first aspect the present invention provides a spontaneously dispersible pharmaceutical composition comprising a 5-aryl-4(R)-arylcarbonylamino-pent-2-enoic acid amide substance P antagonist and a carrier medium comprising a lipophilic component and a surfactant.

The spontaneously dispersible pharmaceutical composition is hereinafter also referred to as a "composition of the invention". It is preferably a microemulsion preconcentrate.

Terms used in the specification have the following meanings:
"Active agent" as used herein means a 5-aryl-4(R)-arylcarbonylamino-pent-2-enoic acid amide substance P antagonist, such as those disclosed in WO 98/07694.
"Poorly water soluble" as used herein means having a solubility in water at 20°C of less than 1%, for example 0.01 % weight/volume, i.e. a "sparingly soluble to very slightly soluble drug" as described in Remington: The Science and Practice of Pharmacy, 19th Edition, Ed. A.R. Gennaro, Mack Publishing Company, US, 1995, vol. 1, p 195.
"Bioavailable" as used herein with reference to a composition means the composition provides a maximum concentration of the active agent in that composition in a use environment that is at least 1.5 fold that of a control comprising an equivalent quantity of the undispersed drug.
"Spontaneously dispersible pharmaceutical composition" as used herein means a composition that contains an active agent herein defined and is capable of producing colloidal structures-when diluted with an aqueous medium, for example water, or in gastric juices. The colloidal structures can be solid or preferably liquid particles including droplets and nanoparticles. The spontaneously dispersible pharmaceutical composition is preferably a microemulsion preconcentrate.
"Microemulsion preconcentrate" as used herein means a composition which spontaneously forms a microemulsion in an aqueous medium, for example, in water, for example on dilution of 1: 1 to 1: 300, preferably 1: 1 to 1: 70, but especially 1: 1 to 1: 10 or in the gastric juices after oral application.
"Microemulsion" as used herein means a slightly opaque, opalescent, non-opaque or substantially non-opaque colloidal dispersion that is formed spontaneously or substantially spontaneously when its components arc brought into contact with an aqueous medium. A microemulsion is thermodynamically stable and typically contains dispersed droplets of a mean diameter less than about 200 nm (2000 A). Generally microcmulsions comprise droplets or liquid nanoparticles that have a mean diameter of less than about 150 nm ( 1500 A); typically less than 100 nm, generally greater than 10 nm, and they are stable over periods up to 24 hours.

Microemulsions offer greater ease of preparation due to spontaneous formation, thermodynamic stability, transparent and elegant appearance, increased drug loading, chanced penetration through the biological membranes, increased bioavailability, and less inter- and intra-individual variability in drug pharmacokinetics than coarse emulsions.

Further characteristics of microcmulsions can be found in United Kingdom patent specification GB 2,222,770; Rosof, Progress in Surface and Membrane Science, 12, 405 et seq. Academic Press (1975); Friberg, Dispersion Science and Technology, 6 (3), 317 et scq. (1985); and Muller et al. Pharm. Ind., 50 (3), 370 et seq. (1988)].

Throughout this specification and in the claims that follow, unless the context requires otherwise, the word "comprise", or variations such as "comprises" or "comprising, will be understood to imply the inclusion of a stated integer or step or group of integers or steps but not the exclusion of any other integer or step or group of integers or steps

In a second aspect the present invention provides a spontaneously dispersible pharmaceutical composition comprising a 5-aryl-4(R)-arylcarbonylamino-pent-2-enoic acid amide substance P antagonist as an active agent, and a carrier medium comprising a lipophilic component, a surfactant, and further a hydrophilic component.

Preferably the spontaneously dispersible pharmaceutical composition is suitable for oral administration.

The 5-aryl-4(R)-arylcarbonylamino-pent-2-enoic acid amide substance P antagonist is poorly water soluble as herein defined. It has a water solubility of below 0.001 %, e.g. 0.001 to 0.0001 %.

The active agent is preferably used in free base form.

In a third aspect the present invention provides a microemulsion preconcentrate comprising a 5-aryl-4(R)-arylcarbonylamino-pent-2-enoic acid amide substance P antagonist and a carrier medium comprising a lipophilic component and a surfactant.

In a fourth aspect the present invention provides a microemulsion preconcentrate comprising a 5-aryl-4(R)-arylcarbonylamino-pent-2-enoic acid amide substance P antagonist and a carrier medium that comprises a lipophilic component, a surfactant, and further a hydrophilic component.

The microemulsion preconcentrate preferably forms an o/w (oil-in-water) microemulsion when diluted with water.

Preferably the relative proportions of the lipophilic component(s), the hydrophilic components), and the surfactant(s) lie within the "Microemulsion" region on a standard three way plot graph. These phase diagrams, can be generated in a conventional manner as described in e.g. GB 2,222,770 or WO 96/13273.

In a fifth aspect the present invention provides a microemulsion comprising a 5-aryl-4(R)-arylcarbonylamino-pent-2-enoic acid amide substance P antagonists and a carrier medium comprising a lipophilic component and a surfactant.

The microemulsion is preferably an o/w (oil-in-water) microemulsion.

In a sixth aspect the present invention provides a microemulsion comprising a 5-aryl-4(R)-arylcarbonylamino-pent-2-enoic acid amide substance P antagonist, a lipophilic component, a surfactant, water, and further a hydrophilic component.

The colloidal structures of the microemulsion form spontaneously or substantially spontaneously when the components of the composition of the invention are brought into contact with an aqueous medium, e.g. by simple shaking by hand for a short period of time, for example for 10 seconds. The compositions of the invention are thermodynamically stable, e.g. for at least 15 minutes or up to 4 hours, even to 24 hours. Typically, they contain dispersed structures, i.e. droplets or liquid nanoparticles of a mean diameter less than about 200 nm (2,000 A), e.g. less than about 150 nm (1,500 A), typically less than 100 nm (1,000 Å), generally greater than 10 nm (100 A) as measured by standard light scattering techniques, e.g. using a MALVERN ZETASIZER 3000^{™} particle characterising machine.

Solid drug particles of mean diameter greater than 200 nm may also be present. The proportion of particles present may be temperature dependent.

The active agent is a neurokinin antagonist (NK antagonist) and is therefore capable of preventing disease symptoms that are caused *inter alia* by the activation of the NK1 receptor by substance P and the activation of the NK2 receptor by neurokinin A [=NKA]. The substance-P-antagonising effects can be demonstrated, for example, as follows: in vitro, for example, the binding of ³H-substance P to the bovine retina in the radio receptor assay according to H. Bittiger, Ciba Foundation Symposium 91 (1982) 196-199*,* is inhibited with IC₅₀ values of from about 0.2 nM.

More specifically the active agent is a 5-aryl-4(R)-arylcarbonylamino-pent-2-enoic acid amide substance P antagonist. This class of compounds is described in WO 98/07694.

Preferred active agents, which are described in WO 98/07694, include:
(4R)-[N'-methyl-N'-(3,5-bistrifluoromethyl-benzoyl)-amino]-5-(1-methyl-indol-3-yl)-pent-2-enoic acid N-[(R)-epsilon-caprolactam-3-yl]-amide,
(4R)-[N'-methyl-N'-(3,5-bistrifluoromethyl-benzoyl)-amino]-5-(1-methyl-indol-3-yl)-pent-2-enoic acid N-[(S)-epsilon-caprolactam-3-yl]-amide,
(4R)-[N'-methyl-N'-(3,5-bistrifluoromethyl-benzoyl)-amino]-5-(1-methyl-indol-3-yl)-pent-2-enoic acid N-cyclohexyl-amide,
(4R)-[N'-methyl-N'-(3,5-bistrifluoromethyl-benzoyl)-amino]-5-(1-methyl-indol-3-yl)-2-methyl-pent-2-enoic acid N-cyclohexyl-amide,
(4R)-[N'-methyl-N'-(3,5-bistrifluoromethyl-benzoyl)-amino]-5-(1-methyl-indol-3-yl)-2-methyl-pent-2-enoic acid N-[(R)-epsilon-caprolactam-3-yl]-amide,
(4R)-[N'-methyl-N'-(3,5-bistrifluoromethyl-benzoyl)-amino]-5-(1-methyl-indol-3-yl)-2-methyl-pent-2-enoic acid N-[(S)-epsilon-caprolactam-3-yl]- amide,
(4R)-(N'-methyl-N'-benzoyl-amino)-5-(1-methyl-indol-3-yl)-2-methyl-pent-2-enoic acid N-[(R)-epsilon-caprolactam-3-yl]-amide,
(4R)-[N'-methyl-N'-(3,5-bistrifluoromethyl-benzoyl)-amino]-5-(naphth-2-yl)-pent-2-enoic acid N-[(R)-epsilon-caprolactam-3-yl]-amide,
(4R)-(N'-methyl-N'-benzoyl)-amino-5-(naphth-2-yl)-pent-2-enoic acid N-[(R)-epsilon-caprolactam-3-yl]-amide,
(4R)-[N'-methyl-N'-(3,5-bistrifluoromethyl-benzoyl)-amino]-5-(naphth-2-yl)-2-methyl-pent-2-enoic acid N-[(R)-epsilon-caprolactam-3-yl]-amide,
(4R)-[N'-methyl-N'-(3,4,5-trimethoxy-benzoyl)-amino]-5-(naphth-2-yl)-2-methyl-pent-2-enoic acid N-[(R)-epsilon-caprolactam-3-yl]-amide,
(4R)-[N'-methyl-N'-(3,5-bistrifluoromethyl-benzoyl)-amino]-5-(naphth-2-yl)-2-methyl-pent-2-enoic acid N-[(S)-epsilon-caprolactam-3-yl]-amide,
(4R)-[N'-methyl-N'-(3,5-bistrifluoromethyl-benzoyl)-amino]-5-(naphth-2-yl)-2-methyl-pent-2-enoic acid N-cyclohexyl-amide,
(4R)-[N'-methyl-N'-(3,5-bistrifluoromethyl-benzoyl)-amino]-5-(1-methyl-indol-3-yl)-pent-2-enoic acid N-[(S)-epsilon-caprolactam-3-yl]-amide,
(4R)-[N'-methyl-N'-(3,5-bistrifluoromethyl-benzoyl)-amino]-4-(4-chlorobenzyl)-but-2-enoic acid N-[(R)-epsilon-caprolactam-3-yl]-amide,
(4R)-[N'-methyl-N'-(3,5-bistrifluoromethyl-benzoyl)-amino]-4-(4-chlorobenzyl)-but-2-enoic acid N-[(S)-epsilon-caprolactam-3-yl]-amide,
(4R)-[N'-methyl-N'-(3,5-bistrifluoromethyl-benzoyl)-amino]-4-(4-chlorobenzyl)-but-2-enoic acid N-cyclohexyl-amide,
(4R)-[N'-methyl-N'-(3,5-bistrifluoromethyl-benzoyl)-amino]-4-(3,4-dichlorobenzyl)-but-2-enoic acid N-cyclohexyl-amide,
(4R)-[N'-methyl-N'-(3,5-bistrifluoromethyl-benzoyl)-amino]-4-(3,4-difluorobenzyl)-but-2-enoic acid N-cyclohexyl-amide,
(4R)-[N'-methyl-N'-(3,5-bistrifluoromethyl-benzoyl)-amino]-5-(4-chlorophenyl)-2-methyl-pent-2-enoic acid N-cyclohexylamide,
(4R)-[N'-methyl-N'-(3,5-bistrifluoromethyl-benzoyl)-amino]-5-(4-chlorophenyl)-2-methyl-pent-2-enoic acid N-[(R)-epsilon-caprolactam-3-yl]-amide,
(4R)-[N'-methyl-N'-(3,5-bistrifluoromethyl-benzoyl)-amino]-4-(4-chlorobenzyl)-2-methyl-but-2-enoic acid [(S)-epsilon-caprolactam-3-yl]-amide,
(4R)-[N'-ethyl-N'-(3,5-bistrifluoromethyl-benzoyl)-amino]-5-(4-chlorophenyl)-pent-2-enoic acid N-[(S)-epsilon-caprolactam-3-yl]-amide,
(4R)-[N'-methyl-N'-(3,5-bistrifluoromethyl-benzoyl)-amino]-5-(4-chlorophenyl)-3-methyl-pent-2-enoic acid N-cyclohexyl-amide,
(4R)-[N'-methyl-N'-(3,5-bistrifluoromethyl-benzoyl)-amino]-4-(4-chlorobenzyl)-3-methyl-but-2-enoic acid [(R)-epsilon-caprolactam-3-yl]-amide,
(4R)-[N'-methyl-N'-(3,5-bistrifluoromethyl-benzoyl)-amino]-4-(4-chlorobenzyl)-but-2-enoic acid N-[(R)-epsilon-caprolactam-3-yl]-amide,
(4R)-4-[N'-methyl-N'-(3,5-bistrifluoromethyl-berizoyl)-amino]-4-(3,4-dichlorobenzyl)-but-2-enoic acid N-[(R)-epsilon-caprolactam-3-yl]-amide,
(4R)- and (4S)-4-[N'-methyl-N'-(3,5-bistrifluoromethyl-benzoyl)-amino]-4-(3-fluoro-4-chlorobenzyl)-but-2-enoic acid N-[(R)-epsilon-caprolactam-3-yl]-amide,
(4R)- and (4S)-4-[N'-methyl-N'-(3,5-bistrifluoromethyl-benzoyl)-amino]-4-(3,4-difluorobenzyl)-but-2-enoic acid N-[(R)-epsilon-caprolactam-3-yl]-amide,
(4R)- and (4S)-4-[N'-methyl-N'-(3,5-bistrifluoromethyl-benzoyl)-amino]-4-(3,4-dibromobenzyl)-but-2-enoic acid N-[(R)-epsilon-caprolactam-3-yl]-amide,
(4R)- and (4S)-4-[N'-methyl-N'-(3,5-bistrifluoromethyl-benzoyl)-amino]-4-(3,4,5-trifluorobenzyl)-but-2-enoic acid N-[(R)-epsilon-caprolactam-3-yl]-amide,
(4R)- and (4S)-4-[N'-methyl-N'-(3,5-bistrifluoromethyl-benzoyl)-amino]-4-(4-fluorobenzyl)-but-2-enoic acid N-[(R)-epsilon-caprolactam-3-yl]-amide,
(4R)- and (4S)-[N'-(3,5-bistrifluoromethyl-benzoyl)-N'-methyl-amino]-5,5-diphenyl-pent-2-enoic acid N-[(S)-epsilon-caprolactam-3-yl]-amide,
(4S)-4-[N'-methyl-N'-(3,5-bistrifluoromethylbenzoyl)amino]-4-(3,4-dichlorobenzyl)-but-2-enoic acid N-[(R)-epsilon-caprolactam-3-yl]-amide,
(4R)-4-[N'-methyl-N'-(3,S-bistrifluoromethylbenzoyl)amino]-4-(3,4-dichlorobenzyl)-but-2-enoic acid N-[(S)-epsilon-caprolactam-3-yl]-amide, and
(4S)-4-[N'-methyl-N'-(3,5-bistrifluoromethylbenzoyl)amino]-4-(3,4-dichlorobenzyl)-but-2-enoic acid N-[(S)-epsilon-caprolactam-3-yl]-amide.

The especially preferred active agent is (4R)-4-[N'-methyl-N'-(3,5-bistrifluoro-methylbenzoyl)amino]-4-(3,4-dichlorobenzyl)-but-2-enoic acid N-[(R)-epsilon-caprolactam-3-yl]-amide, i.e. a compound of formula I

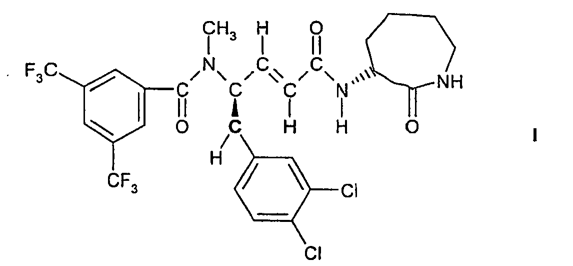

This compound is hereinafter referred to as Compound A.

The preferred and especially preferred active agents, in free or pharmaceutically acceptable salt form, may be prepared as described in international patent application WO 98/07694. As mentioned therein, they may be in the form of their hydrates and/or may include other solvents, for example solvents which may have been used for the crystallisation of compounds in solid form.

In accordance with the present invention the active agent may be preset in an amount by weight of up to about 20% by weight of the composition of the invention, e.g. from about 0.05% by weight. The active agent is preferably present in an amount of 0.5 to 15 % by weight of the composition, more preferably in an amount of 1.5 to 5% by weight of the composition.

The active agent is poorly water soluble so it is carried in a carrier medium.

In the compositions of the invention the carrier medium comprises a lipophilic component and a surfactant. In some embodiments the carrier medium comprises a lipophilic component, a surfactant and further a hydrophilic component.

The lipophilic component comprises one or more lipophilic substances. The hydrophilic component comprises one or more hydrophilic substances. And the carrier medium can contain one or more surfactants.

The compositions of the invention can include a variety of additives including antioxidants, antimicrobial agents, enzyme inhibitors, stabilizers, preservatives, flavours, sweeteners and further components such as those described in Fiedler, H. P. "Lexikon der Hilfsstoffe für Pharmazic, Kosmetik und angrenzende Gebiete", Editio Cantor, D-7960 Aulendorf, 4th revised and expanded edition (1996). These additives will conveniently be dissolved in the carrier medium.

The compositions of the invention include a lipophilic component or phase. The active agent may be contained in this component of the carrier medium.

The lipophilic component is preferably characterized by a low HLB value of less than 10, e.g. up to 8. Suitable lipophilic components include:

### 1) Glyceryl mono-C₆-C₁₄-fatty acid esters

These are obtained esterifying glycerol with vegetable oil followed by molecular distillation. Monoglycerides suitable for use in the compositions of the invention include both symmetric (i.e. β-monoglycerides) as well as asymmetric monoglycerides (α-monoglycerides. They also include both uniform glycerides (in which the fatty acid constituent is composed primarily of a single fatty acid) as well as mixed glycerides (i.e. in which the fatty acid constituent is composed of various fatty acids) The fatty acid constituent may include both saturated and unsaturated fatty acids having a chain length of e.g. C₈-C₁₄. Particularly suitable are caprylic or lauric acid monoglycerides which are commercially available, e.g. under the trade names Imwitor® 308 or Imwitor® 312, respectively, from e.g. sasol. For example Imwitor® 308 comprises at least 80 % monoglycerides and exhibits the following additional characterising data: free glycerol max 6 %, acid value max. 3, saponification value 245-265, iodine value max. 1, water content max. 1 %. Typically it comprises 1 % free glycerol, 90 % monoglycerides, 7 % diglycerides, 1 % triglycerides (H. Fiedler, *loc. cit*., volume 1, page 798). A further example is Capmul MCM C8 from Abitec Corporati on.

### 2) Mixtures of mono- and di-glycerides of C₆-C₁₈ fatty acids

These include both symmetric (i.e. β-monoglycerides and α,α¹-diglycerides) as well as asymmetric mono- and di-glycerides (i.e. α-monoglycerides and α,β-diglycerides) and acetylated derivatives thereof. They also include both uniform glycerides (in which the fatty acid constituent is composed primarily of a single fatty acid) as well as mixed glycerides (i.e. in which the fatty acid constituent is composed of various fatty acids) and any derivatives thereof with lactic or citric acid. The fatty acid constituent may include both saturated and unsaturated fatty acids having a chain length of from e.g. C₈-C₁₀. Particularly suitable are mixed caprylic and capric acid mono- and di-glycerides as commercially available, e.g. under the trade name Imwitor® 742 or Imwitor 928, from e.g. sasol. For example Imwitor® 742 comprises at least 45% monoglycerides and exhibits the following additional characterising data: free glycerol max. 2 %, acid value max. 2, saponification value 250-280, iodine value max. 1, water max. 2 % (H. Fiedler, *loc. cit.*, vol 1, page 798). Other suitable mixtures comprise mono/diglycerides of caprylic/capric acid in glycerol as known and commercially available under e.g. the trade name Capmul® MCM from e.g. Abitec Corporation. Capmul® MCM exhibits the following additional characterising data: acid value 2.5 max., alpha-Mono (as oleate) 80% min., free glycerol 2.5% max., iodine value 1 max., chain length distribution: caproic acid (C6) 3% max., caprylic acid (C8) 75% min., capric acid (C10) 10% min., lauric acid (C12) 1.5% max., moisture (by Karl Fisher) 0.5% max. (manufacturer information). Suitable examples of mono-/di-glcyerides with additional derivatization with lactic or citric acid are those marketed under the brand names of Imwitor 375, 377 or 380 by sasol. Furthermore, the fatty acid constituent may include both saturated and unsaturated fatty acids having a chain length of from e.g. C₁₆-C₁₈. A suitable example is Tegin® O (glyceryl oleate) exhibiting the following additional characterising data: monoglyceride content 55-65%, peroxide value max. 10, water content max. 1%, acid value max. 2, iodine value 70-76, saponification value 158-175, free glycerol max. 2%, (manufacturer information).

### 3) Glyceryl di- C₆-C₁₈-fatty acid esters

These include symmetric (i.e. α,α¹-diglycerides) and asymmetric diglycerides (i.e. α,β-diglycerides) and acetylated derivatives thereof. They also include both uniform glycerides (in which the fatty acid constituent is composed primarily of a single fatty acid) as well as mixed glycerides (i.e. in which the fatty acid constituent is composed of various fatty acids) and any acetylated derivatives thereof. The fatty acid constituent can include both saturated and unsaturated fatty acids having a chain length of from C₆-C₁₈, e.g. C₆-C₁₆, e.g. C₈-C₁₀, e.g. C₈. Particularly suitable is caprylic diglycerides, which is commercially available, e.g. under the trade name Sunfat® GDC-S, e.g. from Taiyo Kagaku Co., Ltd. Sunfat® GDC-S has an acid value of about 0.3, a diglyceride content of about 78.8%, and a monoester content of about 8.9.

### 4) Medium chain fatty acid triglyceride

These include triglycerides of saturated fatty acid having 6 to 12, e.g. 8 to 10, carbon atoms. Suitable medium chain fatty acid triglycerides are those known and commercially available under the trade names Acomed®, Myritol®, Captex®, Neobee®M 5 F, Miglyol®810, Miglyol®812, Miglyol®818, Mazol®, Sefsol®860, Sefsol®870; Miglyol®812 being the most preferred. Miglyol®812 is a fractionated coconut oil comprising caprylic-capric acid triglycerides and having a molecular weight of about 520 Daltons. Fatty acid composition = C₆ max. about 3%, C₈ about 50 to 65%, C₁₀ about 30 to 45%, C₁₂ max 5%; acid value about 0.1; saponification value about 330 to 345; iodine value max 1. Miglyol® 812 is available from Condea. Neobee® M 5 F is a fractionated caprylic-capric acid triglyceride available from coconut oil; acid value max. 0.2; saponification value about 335 to 360; iodine value max 0.5, water content max. 0,15%, D.²⁰ 0,930-0,960, n_{D}²⁰ 1,448-1,451 (manufacturer information). Neobee® M 5 F is available from Stepan Europe. A further example is Miglyol 829 containing additionally esters with succinic acid.

### 5) Glyceryl mono-C₁₆-C₁₈-fatty acid esters

These are obtained esterifying glycerol with vegetable oil followed by molecular distillation. Monoglycerides suitable for use in the compositions of the invention include both symmetric (i.e. β-monoglycerides) as well as asymmetric monoglycerides (α-monoglycerides. They also include both uniform glycerides (in which the fatty acid constituent is composed primarily of a single fatty acid) as well as mixed glycerides (i.e. in which the fatty acid constituent is composed of various fatty acids) The fatty acid constituent may include both saturated and unsaturated fatty acids having a chain length of from e.g. C₁₆-C₁₈. Suitable examples include GMOrphic by Estman, Rylo MG20 distilled monoglyceride by Danisco Ingredients, or Monomuls 90-018 by Henkel. For example GMOrphic®-80 (glyceryl monooleate) exhibits the following additional characterising data: monoglyceride content min. 94%, C18:1 content 75% min., peroxide value max. 2.5, C18:2 + C18:3 max. 15%, C16:0 + C18: 0 + C20:0 max. 10%, water max. 2%, acid value max. 3, iodine value 65-75, saponification value 155-165, free glycerine max. 1%, hydroxyl number 300-330 (manufacturer information).

### 6) Mixed mono-, di-, tri-glycerides

These include mixed mono-, di-, tri-glycerides that are commercially available under the trade name Maisine® from Gattefossé. They are transesterification products of corn oil and glycerol. Such products are comprised predominantly of linoleic and oleic acid mono-, di- and tri-glycerides together with minor amounts of palmitic and stearic acid mono-, di- and tri-glycerides (corn oil itself being comprised of ca. 56% by weight linoleic acid, 30% oleic acid, ca. 10% palmitic and ca. 3% stearic acid constituents). Physical characteristics are: free glycerol max 10%, monoglycerides ca. 40%, diglycerides ca. 40%, triglycerides ca. 10%, free oleic acid content ca. 1%. Further physical characteristics are: acid value max. 2, iodine value of 85-105, saponification value of 150-175, mineral acid content = 0. The fatty acid content for Maisine® is typically: palmitic acid ca. 11%, stearic acid ca. 2.5%, oleic acid ca. 29%, linoleic acid ca. 56%, others ca. 1.5% (H. Fiedler, *loc. cit.,* volume 2, page 958; manufacturer information).

Mixed mono-, di-, tri-glycerides preferably comprise mixtures of C₈ to C₁₀ or C₁₂₋₂₀ fatty acid mono-, di- and tri-glycerides, especially mixed C₁₆₋₁₈ fatty acid mono-, di- and triglycerides. The fatty acid component of the mixed mono-, di- and tri-glycerides may comprise both saturated and unsaturated fatty acid residues. Preferably however they are predominantly comprised of unsaturated fatty acid residues; in particular C₁₈ unsaturated fatty acid residues. Suitably the mixed mono-, di-, tri-glycerides comprise at least 60%, preferably at least 75%, more preferably at least 85% by weight of a C₁₈ unsaturated fatty acid (for example linolenic, linoleic and oleic acid) mono-, di- and tri-glycerides. Suitably the mixed mono-, di-, tri-glycerides comprise less than 20%, for example about 15% or 10% by weight or less, saturated fatty acid (for example palmitic and stearic acid) mono-, di- and tri-glycerides. Mixed mono-, di-, tri-glycerides are preferably predominantly comprised of mono- and di-glycerides; for example mono- and di-glycerides comprise at least 50%, more preferably at least 70% based on the total weight of the lipophilic phase or component. More preferably, the mono- and di-glycerides comprise at least 75% (for example about 80% or 85% by weight of the lipophilic component. Preferably monoglycerides comprise from about 25 to about 50%, based on the total weight of the lipophilic component, of the mixed mono-, di-, tri-glycerides. More preferably from about 30 to about 4-0% (for example 35 to 40%) monoglycerides are present. Preferably diglycerides comprise from about 30 to about 60%, based on the total weight of the lipophilic component, of the mixed mono-, di-, tri-glycerides. More preferably from about 40 to about 55% (for example 48 to 50%) diglycerides are present. Triglycerides suitably comprise at least 5% but less than about 25 %, based on the total weight of the lipophilic component, of the mixed mono-, di-, tri-glycerides. More preferably from about 7.5 to about 15% (for example from about 9 to 12%) triglycerides are present. Mixed mono-, di-, tri-glycerides may be prepared by admixture of individual mono-, di- or tri-glycerides in appropriate relative proportion. Conveniently however they comprise transesterification products of vegetable oils, for example almond oil, ground nut oil, olive oil, peach oil, palm oil or, preferably, corn oil, sunflower oil or safflower oil and most preferably corn oil, with glycerol. Such transesterification products are generally obtained as described in GB 2 257 359 or WO 94/09211. Preferably some of the glycerol is first removed to give a "substantially glycerol free batch" when soft gelatine capsules are to be made. Purified transesterification products of corn oil and glycerol provide particularly suitable mixed mono-, di-, and tri-glycerides hereinafter referred to as "refined oil" and produced according to procedures described in United Kingdom patent specification GB 2,257,359 or international patent publication WO 94/09211.

### 7) Acetylated monoglycerides (C18)

These include Myvacet 9-45.

### 8) Propylene glycol monofatty acid esters

The fatty acid constituent may include both saturated and unsaturated fatty acids having a chain length of from e.g. C₈-C₁₂. Particularly suitable are propylene glycol mono ester of caprylic and lauric acid as commercially available, e.g. under the trade names Sefsol® 218, Capryol®90 or Lauroglycol®90, from e.g. Nikko Chemicals Co., Ltd. or Gattefossé or Capmul PG-8 from Abitec Corporation. For example Lauroglycol®90 exhibits the following additional characterising data: acid value max. 8, saponification value 200-220, iodine value max. 5, free propylene glycol content max. 5%, monoester content min. 90%; Sefsol® 218 exhibits the following additional characterising data: acid value max. 5, hydroxy value 220-280 (H. Fiedler, *loc, cit*., vol 2, page 906, manufacturer information).

### 9) Propylene glycol mono- and di- fatty acid esters

These include Laroglycol FCC and Capryol PGMC.

### 10) Propylene glycol diesters

Propylene glycol di-fatty acid esters such as propylene glycol dicaprylate (which is commercially available under the trade name Miglyol® 840 from e.g. sasol; H. Fiedler, *loc. cit*., volume 2, page 1008) or Captex 200 from Abitec Corporation.

### 11) Propylene glycol monoacetate and Propylene glycol diacetate

### 12) Transesterified ethoxylated vegetable oils

These include transesterified ethoxylated vegetable oils such as those obtained by reacting various natural vegetable oils (for example, maize oil, kernel oil, almond oil, ground nut oil, olive oil, soybean oil, sunflower oil, safflower oil and palm oil, or mixtures thereof) with polyethylene glycols that have an average molecular weight of from 200 to 800, in the presence of an appropriate catalyst. These procedures are described in United States patent specification US 3,288,824. Transesterified ethoxylated corn oil is particularly preferred.

Transesterified ethoxylated vegetable oils are known and are commercially available under the trade name Labrafil® (H. Fiedler, loc. cit., vol 2, page 880). Examples are Labrafil® M 2125 CS (obtained from corn oil and having an acid value of less than about 2, a saponification value of 155 to 175, an HLB value of 3 to 4, and an iodine value of 90 to 110), and Labrafil® M 1944 CS (obtained from kernel oil and having an acid value of about 2, a saponification value of 145 to 175 and an iodine value of 60 to 90). Labrafil® M 2130 CS (which is a transesterification product of a C₁₂₋₁₈ glyceride and polyethylene glycol and which has a melting point of about 35 to 40°C, an acid value of less than about 2, a saponification value of 185 to 200 and an iodine value of less than about 3) may also be used. The preferred transesterified ethoxylated vegetable oil is Labrafil® M 2125 CS which can be obtained, for example, from Gattefossé, Saint-Priest Cedex, France.

### 13) Sorbitan fatty acid esters

Such esters include e.g. sorbitan mono C₁₂₋₁₈ fatty acid esters, or sorbitan tri C₁₂₋₁₈ fatty acid esters are commercially available under the trade mark Span® from e.g. uniqema. An especially preferred product of this class is e.g. Span® 20 (sorbitan monolaurate) or Span® 80 (sorbitan monooleate) (Fiedler, loc. cit., 2, p. 1430; Handbook of Pharmaceutical Excipients, loc. cit., page 473).

### 14) Esterified compounds of fatty acid and primary alcohols

These include esterified compounds of fatty acid having 8 to 20 carbon atoms and primary alcohol having 2 to 3 carbon atoms, for example, isopropyl myristate, isopropyl palmitatc, ethyl linoleate, ethyl olcate, ethylmyristate etc., with an esterified compound of linoleic acid and ethanol being particularly preferable, also isopropylmyristat and isopropylpalmitat.

### 15) Glycerol triacetate or (1,2,3)-triacetin

This is obtained by esterifying glycerin with acetic anhydride. Glycerol triacetate is commercially available as, e.g. Priacetin® 1580 from Unichema International, or as Eastman™ Triacetin from Eastman, or from Courtaulds Chemicals Ltd. Glycerol triacetate exhibits the following additional characterising data: molecular weight 218,03, D.^{20,3} 1,159-1,163, n_{D}²⁰ 1,430-1,434, water content max. 0.2 %, viscosity (25°) 17.4 mPa s, acid value max. 0.1, saponification value of about 766-774, triacetin content 97% min. (H. Fiedler, loc. cit., vol 2, page 1580; Handbook of Pharmaceutical Excipients, loc. cit., page 534, manufacturer information).

### 16) Acetyl triethyl citrate

This is obtained by esterification of citric acid and ethanol, followed by acetylation with acetic anhydride, respectively. Acetyl triethyl citrate is commercially available, e.g. under the trade name Citroflex® A-2, from e.g. Morflex Inc.

### 17) Tributylcitrate or acetyl tributyl citrate

### 18) Polyglycerol fatty acid esters

These have for example from 2 to 10, e.g. 6 glycerol units. The fatty acid constituent can include both saturated and unsaturated fatty acids having a chain length of from e.g. C₈-C₁₈. Particularly suitable is e.g. Plurol Oleique CC497 from Gattefossé, having a saponification value of 133-155 and a saponification value of 196-244. Further suitable polyglycerol fatty acid esters include diglyceryl monooleate (DGMO) and Hexaglyn-5-O, as known and commercially available from e.g. Nikko Chemicals Co., Ltd.

### 19) PEG-fatty alcohol ether

This includes Brij 30™ polyoxyethylene(4) lauryl ether.

### 20) Fatty alcohols and fatty acids

Fatty acids can be obtained by hydrolysing various animal and vegetable fats or oils, such as olive oil, followed by separation of the liquid acids. The fatty acid/alcohol constituent can include both saturated and mono- or di-unsaturated fatty acids/alcohols having a chain length of from e.g. C₆-C₂₀. Particularly suitable are, e.g. oleic acid, oleyl alcohol, linoleic acid, capric acid, caprylic acid, caproic acid, tetradecanol, dodecanol, or decanol. Oleyl alcohol is commercially available under the trade mark HD-Eutanol® V from e.g. Henkel KGaA. Oleyl alcohol exhibits the following additional characterising data: acid value max 0.1, hydroxy value of about 210, iodine value of about 95, saponification value max 1, D.²⁰ about 0,849, n_{D}²⁰ 1,462, molecular weight 268, viscosity (20°) about 35 mPa s (manufacturer information). Oleic acid exhibits the following additional characterising data: molecular weight 282,47, D.²⁰ 0,895, n_{D}²⁰ 1,45823, acid value 195-202, iodine value 85-95, viscosity (25°) 26 mPa s (H. Fiedler, loc. cit., volume 2, page 1112; "Handbook of Pharmaceutical Excipients", 2nd Edition, Editors A. Wade and P. J. Weller (1994), Joint publication of American Pharmaceutical Assoc., Washington, USA and The Pharmaceutical Press, London, England, page 325).

### 21) Tocopherol and its derivatives (e.g. acetate)

These include Coviox T-70, Copherol 1250, Copherol F-1300, Covitol 1360 and Covitol 1100.

### 22) Pharmaceutically acceptable oils

Alternatively the lipophilic component comprises e.g. a pharmaceutically acceptable oil, preferably with an unsaturated component such as a vegetable oil.

### 23) Alkylene polyol ethers or esters

These include C₃₋₅alkylene triols, in particular glycerol, ethers or esters. Suitable C₃₋₅alkylene triol ethers or esters include mixed ethers or esters, i.e. components including other ether or ester ingredients, for example transesterification products of C₃₋₅alkylene triol esters with other mono-, di- or poly-ols. Particularly suitable alkylene polyol ethers or esters are mixed C₃₋₅alkylene triol/poly-(C₂₋₄alkylene) glycol fatty acid esters, especially mixed glycerol/polyethylene- or polypropylene-glycol fatty acid esters. Especially suitable alkylene polyol ethers or esters include products obtainable by transesterification of glycerides, e.g. triglycerides, with poly-(C₂-₄alkylene) glycols, e.g. poly-ethylene glycols and, optionally, glycerol. Such transesterification products are generally obtained by alcoholysis of glycerides, e.g. triglycerides, in the presence of a poly-(C-₂₋₄alkylene) glycol, e.g. polyethylene glycol and, optionally, glycerol (i.e. to effect transesterification from the glyceride to the poly-alkylene glycol/glycerol component, i.e. via poly-alkylene glycolysis/glycerolysis). In general such reaction is effected by reacting the indicated components (glyceride, polyalkylene glycol and, optionally, glycerol) at elevated temperature under an inert atmosphere with continuous agitation.

Preferred glycerides are fatty acid triglycerides, e.g. (C₁₀₋₂₂fatty acid) triglycerides, including natural and hydrogenated oils, in particular vegetable oils. Suitable vegetable oils include, for example, olive, almond, peanut, coconut, palm, soybean and wheat germ oils and, in particular, natural or hydrogenated oils rich in (C₁₂₋₁₈fatty acid) ester residues. Preferred polyalkylene glycol materials are polyethylene glycols, in particular polyethylene glycols having a molecular weight of from ca. 500 to ca. 4,000, e.g. from ca. 1,000 to ca. 2,000.

Suitable alkylene polyol ethers or esters include mixtures of C₃₋₅alkylene triol esters, e.g. mono-, di- and tri-esters in variable relative amount, and poly (C₂₋₄alkylene) glycol mono- and di-esters, together with minor amounts of free C₃₋₅alkylene triol and free poly-(C₂₋₅alkylene) glycol. As hereinabove set forth, the preferred alkylene triol moiety is glyceryl; preferred polyalkylene glycol moieties include polyethylene glycol, in particular having a molecular weight of from ca. 500 to ca. 4,000; and preferred fatty acid moieties will be C₁₀₋₂₂fatty acid ester residues, in particular saturated C₁₀₋₂₁fatty acid ester residues.

Particularly suitable alkylene polyol ethers or esters include transesterification products of a natural or hydrogenated vegetable oil and a polyethylene glycol and, optionally, glycerol; or compositions comprising or consisting of glyceryl mono-, di- and tri-C₁₀₋₂₂fatty acid esters and polyethylene glycol mono- and di-C₁₀₋₂₂fatty esters (optionally together with, e.g. minor amounts of free glycerol and free polyethylene glycol).

Preferred vegetable oils, polyethylene glycols or polyethylene glycol moieties and fatty acid moieties in relation to the above definitions are as hereinbefore set forth.

Particularly suitable alkylene polyol ethers or esters as described above for use in the present invention include those commercially available under the trade name Gelucire® from e.g. Gattefossé, in particular the products:
a) Gelucire® 33/01, which has an m.p. = ca. 33-37°C and a saponification value of ca. 230-255;
b) Gelucire® 39/01, m.p. = ca. 37.5-41.5°C, saponification v. = ca. 225-245;
c) Gelucire® 43/01, m.p. = ca. 42-46°C, saponification v. = ca. 220-240;

Products (a) to (c) above all have an acid value of maximum of 3. The compositions of the invention may include mixtures of such ethers or esters.

### 24) Hydrocarbons

These include e.g. squalene, available from e.g. Nikko Chemicals Co., Ltd.

### 25) Ethylene glycol esters

These include Monthyle® (ethylene glycol monostearate), available from e.g. Gattefossé.

### 26) Pentaerythriol fatty acid esters and polyalkylene glycol ethers

These include, for example pcntaerythrite-diolcate, -distearate, -monolaurate, -polyglycol ether, and -monostearate as well as pentaerythrite-fatty acid esters (Fiedler, loc. cit., 2, p. 1158-1160).

Some of these, e.g. (1-3, 5-6, 8-9, 12-13, 19), display surfactant-like behaviour and may also be termed eo-surfactartts.

The lipophilic component preferably comprises 5 to 85 % by weight, of the composition of the invention, e.g. 10 to 85%; preferably 15 to 60 % by weight, more preferably about 20 to about 40 % by weight.

For these embodiments where the carrier medium comprises a hydrophilic component in addition to the lipophilic component and the surfactant the relative proportions of the lipophilic component(s), hydrophilic component(s) and the surfactant(s) lie within the "Microemulsion" region on a standard three way plot graph.

The compositions of the invention may include a hydrophilic component or phase.

Suitable hydrophilic compounds include:

### 1) Polyethylene glycol glyceryl C₆₋C₁₀ fatty acid esters

The fatty acid ester may include mono and/or di and/or tri fatty acid esters. It optionally includes both saturated and unsaturated fatty acids having a chain length of from e.g. C₈-C₁₀. The polyethylene glycols may have e.g. from 5 to 10 [CH₂-CH₂-O] units, e.g. 7 units. A particularly suitable fatty acid ester is polyethylene glycol (7) glyceryl monococoate, which is commercially available, e.g. under the trade name Cetiol® HE, e.g. from Henkel KGaA. Cetiol® HE has a D. (20°) of 1,05, an acid value of less than 5, a saponification value of about 95, a hydroxyl value of about 180 and an iodine value of less than 5 (H. Fiedler, loc. cit., vol 1, page 337) or Lipestrol E-810.

### 2) N-alkylpyrrolidone

Particularly suitable is, e.g. N-Methyl-2-pyrrolidone, e.g. as commercially available under the trade name Pharmasolve™, from e.g. International Specialty Products (ISP). N-methylpyrrolidone exhibits the following additional characterising data: molecular weight 99,1, D.²⁵ 1,027-1,028, purity (as area % by GC) (including Methyl Isomers) 99.85% min (H. Fiedler, loc. cit., vol 2, page 1004, manufacturer information).

### 3) Benzyl alcohol

This is commercially available from e.g. Merck or may be obtained by distillation of benzyl chloride with potassium or sodium carbonate. Benzyl alcohol exhibits the following additional characterising data: molecular weight 108,14, D. 1,043-1,049, n_{D} 1,538-1,541. (H. Fiedler, loc. cit., vol 1, page 238; Handbook of Pharmaceutical Excipients, loc. cit., page 35).

### 4) Triethyl citrate

It is obtained esterifying citric acid and ethanol. Triethyl citrate is commercially available, e.g. under the trade names Citroflex® 2, or in a pharmaceutical grade under the name TEC-PG/N, from e.g. Morflex Inc. Particularly suitable is triethyl citrate which has molecular weight of 276,3, a specific gravity of 1,135-1,139, a refractive index of 1,439-1,441, a viscosity (25°) of 35,2 mPa s, assay (anhydrous basis) 99,0-100,5 %, water max. 0,25 % (Fiedler, H. P., loc. cit.,, vol 1, page 371; "Handbook of Pharmaceutical Excipients", loc. cit., page 540).

Other suitable hydrophilic compounds include transcutol (C₂Hs-[O-(CH₂)₂]₂-OH), glycofurol (also known as tetrahydrofurfuryl alcohol polyethylene glycol ether), 1,2-propylene glycol, dimethylisosorbide (Arlasolve), polyethylene glycol, triethylenglycol, ethylacetate, and ethyllactate.

The hydrophilic component may comprise 5 to 60 % by weight of the composition of the invention, e.g. 10 to 50%; preferably 10 to 40 % by weight, more preferably about 10 to about 30% by weight.

The hydrophilic component may comprise a mixture of two or more hydrophilic components.

The compositions of the invention may lower alkanols such as ethanol as a hydrophilic co-component. While the use of ethanol in the compositions is not essential, it has been found to be of particular advantage when the compositions arc to be manufactured in soft gelatine, encapsulated form. This is because storage characteristics arc improved, in particular the risk of active agent precipitation following encapsulation procedures is reduced. Thus the shelf life stability may be extended by employing ethanol or some other such co-component as an additional ingredient of the composition. The ethanol may comprise 0 to 60 % by weight of the composition; preferably 5 to about 30% by weight and more preferably about 5 to 20 % by weight.

The compositions of the present invention contain one or more surfactants to reduce the interfacial tension thereby providing thermodynamic stability.

Surfactants may be complex mixtures containing side products or unreacted starting products involved in the preparation thereof, e.g. surfactants made by polyoxyethylation may contain another side product, c.g. polyethylene glycol. The or each surfactant preferably has a hydrophilic-lipophilic balance (HLB) value of 8 to 17, especially 10 to 17. The HLB value is preferably the mean HLB value.

Suitable surfactants include:

### 1) Reaction products of a natural or hydrogenated castor oil and ethylene oxide

The natural or hydrogenated castor oil may be reacted with ethylene oxide in a molar ratio of from about 1:35 to about 1:60, with optional removal of the polyethylene-glycol component from the products. Various such surfactants are commercially available. Particularly suitable surfactants include polyethyleneglycol-hydrogenated castor oils available under the trade name Cremophor®; Cremophor® RH 40, which has a saponification value of about 50 to 60, an acid value less than about 1, a water content (Fischer) less than about 2%, an n_{D}⁶⁰ of about 1.453-1.457 and an HLB of about 14-16; and Cremophor® RH 60, which has a saponification value of about 40-50, an acid value less than about 1, an iodine value of less than about 1, a water content (Fischer) of about 4.5-5.5%, an n_{D}⁶⁰ of about 1.453-1.457 and an HLB of about 15 to 17.

An especially preferred product of this class is Cremophor® RH40. Other useful products of this class are available under the trade names Nikkol® (e.g. Nikkol®

HCO-40 and HCO-60), Mapeg® (e.g. Mapeg® CO-40h), Incrocas® (e.g. Incrocas® 40), Tagat® (for example polyoxyethylene-glycerol-fatty acid esters e.g. Tagat® RH 40) and Simulsol OL-50 (PEG-40 castor oil, which has a saponification value of about 55 to 65, an acid value of max. 2, an iodine value of 25 to 35, a water content of max. 8%, and an HLB of about 13, available from Seppic). These surfactants are further described in Fiedler *loc. cit*.

Other suitable surfactants of this class include polyethyleneglycol castor oils such as that available under the trade name Cremophor® EL, which has a molecular weight (by steam osmometry) of about 1630, a saponification value of about 65 to 70, an acid value of about 2, an iodine value of about 28 to 32 and an n_{D}²⁵ of about 1.471.

### 2) Polyoxyethylene-sorbitan-fatty acid esters

These include mono- and tri-lauryl, palmityl, stearyl and oleyl esters of the type known and commercially available under the trade name Tween® (Fiedler, *loc. cit*. p.1615 ff) from Uniqema including the products:
Tween® 20 [polyoxyethylene(20)sorbitanmonolaurate],
Tween® 21 [polyoxycthylene(4)sorbitanrnorlolaurate],
Tween® 40 [polyoxyethylene(20)sorbitanmonopalmitatc],
Tween® 60 [polyoxyethylenc(20)sorbitanmonostcarate],
Tween® 65 [polyoxyethylene(20)sorbitantristearate],
Tween® 80 [polyoxyethylene(20)sorbiknmonoolcate],
Tween® 81 [polyoxyethylene(5)sorbitanmonooleate], and
Tween® 85 [polyoxyethylene(20)sorbitantrioleate].

Especially preferred products of this class are Tween® 20 and Tween® 80.

### 3) Polyoxyethylene fatty acid esters

These include polyoxyethylene stearic acid esters of the type known and commercially available under the trade name Myrj® from Uniqema (Fiedler, *loc. cit*., 2, p. 1042). An especially preferred product of this class is Myrj® 52 having a D²⁵ of about 1.1., a melting point of about 40 to 44°C, an HLB value of about 16.9., an acid value of about 0 to I and a saponification no. of about 25 to 35.

### 4) Polyoxyethylene-Rolypxypropylene co-polymers and block co-polymers or poloxamers

These include the type known and commercially available under the trade names Pluronic® and Emkalyx® (Fiedler, *loc. cit.,* 2, p. 1203). An especially preferred product of this class is Pluronic® F68 (poloxamer 188) from BASF, having a melting point of about 52°C and a molecular weight of about 6800 to 8975. A further preferred product of this class is Synpcronic® PE L44 (poloxamer 124) from Uniqema.

### 5) Polyoxyethylene mono esters of a saturated C₁₀ to C₂₂

These include C₁₈ substituted e.g. hydroxy fatty acid; e.g. 12 hydroxy stearic acid PEG ester, e.g. of PEG about e.g. 600-900 e.g. 660 Daltons MW, e.g. Solutol® HS 15 from BASF, Ludwigshafen, Germany. According to the BASF technical leaflet MEF 151E (1986) comprises about 70% polyethoxylated 12-hydroxystcarate by weight and about 30% by weight unesterified polyethylene glycol component. Solutol HS 15 has a hydrogenation value of 90 to 110, a saponification value of 53 to 63, an acid number of maximum 1, and a maximum water content of 0.5% by weight.

### 6) Polyoxyethylene alkyl, ethers

These include polyoxyethylene glycol ethers of C₁₂ to C₁ₓ alcohols, e.g. Polyoxyl 2-, 10- or 20-cctyl ether or Polyoxyl 23-lauryl ether, or polyoxyl 20-oleyl other, or Polyoxyl 2-, 10-, 20-or 100-stearyl ether, as known and commercially available e.g. under the trade mark Brij® from Uniqema. An especially preferred product of this class is e.g. Brij® 35 (Polyoxyl 23 lauryl ether) or Brij® 98 (Polyoxyl 20 oleyl ether) (Fiedler, *loc. cit*., 1, pp. 259; Handbook of Pharmaceutical Excipients, *loc. cit*., page 367). Similarly suitable products include polyoxyethylene-polyoxypropylene-alkyl ethers, e.g. polyoxyethylene-polyoxypropylene-ethers of C₁₂ to C₁₈ alcohols, e.g. polyoxyethylen-20-polyoxypropy-lene-4-cetylether which is known and commercially available under the trade mark Nikkol PBC® 34, from e.g. Nikko Chemicals Co., Ltd. (Fiedler, loc. cit., vol. 2, pp. 1239). Polyoxypropylene fatty acid ethers, e.g. Acconon® E are also suitable.

### 7) Sodium alkyl sulfates and sulfonates, and sodium alkyl aryl sulfonates

These include sodium lauryl sulfate, which is also known as sodium dodecyl sulfate and commercially available, e.g. under the trade name Texapon K12® from Henkel KGaA.

### 8) Water soluble tocopheryl polyethylene glycol succinic acid esters (TPGS)

These include those with a polymerisation number ca 1000, e.g. available from Eastman Fine Chemicals Kingsport, Texas, USA.

### 9) Polyglycerol fatty acid esters

These include those with e.g. from 10 to 20, e.g. 10 glycerol units. The fatty acid constituent may include both saturated and unsaturated fatty acids having a chain length of from e.g. C₈-C₁₈. Particularly suitable is e.g. decaglycerylmonolaurat or decaglycerylmonomyristat, as known and commercially available under the trade mark Decaglyn® 1-L or Decaglyn® 1-M or Decaglyn 1-O, respectively, from e.g. Nikko Chemicals C., Ltd (Fiedler, loc. cit., vol. 2, pp. 1228).

### 10) Alkylene polyol ethers or esters

These include C₃₋₅alkylene triols, in particular glycerol, ethers or esters. Suitable C₃₋₅alkylene triol ethers or esters include mixed ethers or esters, i.e. components including other ether or ester ingredients, for example transesterification products of C₃₋₅alkylene triol esters with other mono-, di- or poly-ols. Particularly suitable alkylene polyol ethers or esters are mixed C₃₋₅alkylene triol/poly-(C₂₋₄alkylene) glycol fatty acid esters, especially mixed glycerol/polyethylene- or polypropylene-glycol fatty acid esters.

Especially suitable alkylene polyol ethers or esters include products obtainable by transesterification of glycerides, e.g. triglycerides, with poly-(C₂₋₄alkylene) glycols, e.g. poly-ethylene glycols and, optionally, glycerol.

Such transesterification products are generally obtained by alcoholysis of glycerides, e.g. triglycerides, in the presence of a poly-(C-₂₋₄alkylene) glycol, e.g. polyethylene glycol and, optionally, glycerol (i.e. to effect transesterification from the glyceride to the poly-alkylene glycol/glycerol component, i.e. via poly-alkylene glycolysis/glycerolysis). In general such reaction is effected by reacting the indicated components (glyceride, polyalkylene glycol and, optionally, glycerol) at elevated temperature under an inert atmosphere with continuous agitation.

Preferred glycerides are fatty acid triglycerides, e.g. (C₁₀₋₂₂fatty acid) triglycerides, including natural and hydrogenated oils, in particular vegetable oils. Suitable vegetable oils include, for example, olive, almond, peanut, coconut, palm, soybean and wheat germ oils and, in particular, natural or hydrogenated oils rich in (C₁₂₋₁₈fatty acid) ester residues.

Preferred polyalkylene glycol materials are polyethylene glycols, in particular polyethylene glycols having a molecular weight of from ca. 500 to ca. 4,000, e.g. from ca. 1,000 to ca. 2,000.

Suitable alkylene polyol ethers or esters include mixtures of C₃₋₅alkylene triol esters, e.g. mono-, di- and tri-esters in variable relative amount, and poly (C₂₋₄alkylene) glycol mono- and di-esters, together with minor amounts of free C₃₋₅alkylene triol and free poly-(C₂₋₅alkylene) glycol. As hereinabove set forth, the preferred alkylene triol moiety is glyceryl; preferred polyalkylene glycol moieties include polyethylene glycol, in particular having a molecular weight of from ca. 500 to ca. 4,000; and preferred fatty acid moieties will be C₁₀₋₂₂fatty acid ester residues, in particular saturated C₁₀₋₂₂fatty acid ester residues.

Particularly suitable alkylene polyol ethers or esters include transesterification products of a natural or hydrogenated vegetable oil and a polyethylene glycol and, optionally, glycerol; or compositions comprising or consisting of glyceryl mono-, di- and tri-C₁₀₋₂₂fatty acid esters and polyethylene glycol mono- and di- C₁₀₋₂₂fatty esters (optionally together with, e.g. minor amounts of free glycerol and free polyethylene glycol).

Preferred vegetable oils, polyethylene glycols or polyethylene glycol moieties and fatty acid moieties in relation to the above definitions are as hereinbefore set forth.

Particularly suitable alkylene polyol ethers or esters as described above for use in the present invention include those commercially available under the trade name Gelucire® from e.g. Gattefossé, in particular the products:
a) Gelucire® 44/14, m.p. = ca. 42.5-47.5°C, saponification v. = ca. 79-93;
b) Gelucire® 50/13, m.p. = ca. 46-51°C, saponification v. = ca. 67-81;

Products (a) to (b) above all have an acid value of maximum of 2.

Alkylene polyol ethers or esters having an iodine value of maximum 2 are generally preferred. The compositions of the invention may include mixtures of such ethers or esters.

Gelucire® products are inert semi-solid way materials with amphiphilic character. They are identified by their melting point and their HLB value. Most Gelucire® grades are saturated polyglycolised glycerides obtainable by polyglycolysis of natural hydrogenated vegetable oils with polyethylene glycols. They are composed of a mixture of mono-, di- and tri-glycerides and mono- and di-fatty acid esters of polyethylene glycol. Particularly suitable is Gelucire® 44/14 which has a nominal melting point of 44°C and an HLB of 14. It is obtained by reacting hydrogenated palm kernels and/or hydrogenated palm oils with polyethylene glycol 1500. It consists of approximately 20 % mono-, di- and triglycerides, 72 % mono- and di- fatty acid esters of polyethylene glycol 1500 and 8% of free polyethylene glycol 1500. The fatty acid distribution for Gelucire® 44/14 is as follows: 4-10 C₈, 3-9 C₁₀, 40-50 C₁₂, 14-24 C₁₄, 4-14 C₁₆, 5-15 C₁₈. Gelucire® 44/14 exhibits the following additional characterising data: acid value of max. 2, iodine value of max. 2, saponification value of 79-93, hydroxyl value of 36-56, peroxide value of max. 6, alkaline impurities max. 80, water content max. 0.50, free glycerol content max. 3, monoglycerides content 3.0-8.0. (H. Fiedler, loc. cit., vol 1, page 676; manufacturer information).

### 11) Polyethylene glycol glyceryl fatty acid esters

The fatty acid ester may include mono and/or di and/or tri fatty acid ester. The fatty acid constituent may include both saturated and unsaturated fatty acids having a chain length of from e.g. C₁₂-C₁₈. The polyethylene glycols may have e.g. from 10 to 40 [CH₂-CH₂-O] units, e.g. 15 or 30 units. Particularly suitable is polyethylene glycol (15) glyceryl monostearat which is commercially available, e.g. under the trade name TGMS®-15, e.g. from Nikko Chemicals Co., Ltd. Other suitable glyceryl fatty acid esters include polyethylene glycol (30) glyceryl monooleate which is commercially available, e.g. under the trade name Tagat® O, e.g. from Goldschmidt (H. Fiedler, loc. cit., vol. 2, p. 1502-1503), and Tagat 02 (polytheylene glycol (20) glycerol monooleate, as well as Tagat L (polytheylene glycol (30) glycerol monolaurate) and Tagat L2 (polytheylene glycol (20) glycerol monolaurate), all e.g. from Goldschmidt (H. Fiedler, loc. cit., vol. 2, p. 1502-1503). A further suitable polyethylene glycol glyceryl fatty acid ester is Tagat TO.

### 12) Sterols and derivatives thereof

These include cholesterols and derivatives thereof, in particular phytosterols, e.g. products comprising sitosterol, campesterol or stigmasterol, and ethylene oxide adducts thereof, for example soya sterols and derivatives thereof, e.g. polyethylene glycol sterols, e.g. polyethylene glycol phytosterols or polyethylene glycol soya sterols. The polyethylene glycols may have e.g. from 10 to 40 [CH₂-CH₂-O] units, e.g. 25 or 30 units. Particularly suitable is polyethylene glycol (30) phytosterol which is commercially available, e.g. under the trade name Nikkol BPS®-30, e.g. from Nikko Chemicals Co., Ltd. Further suitable is polyethylene glycol (25) soya sterol which is commercially available, e.g. under the trade name General® 122 E 25, e.g. from Henkel (H. Fiedler, loc. cit., vol. 1, p. 680).

### 13) Transesterified, polyoxyethylated caprylic-capric acid glycerides

These include those that are commercially available under the trade name Labrasol® from e.g. Gattefossé. Labrasol® has an acid value of max. 1, a saponification value of 90-110, and an iodine value of max. 1 (H. Fiedler, loc. cit., vol 2, page 880).

### 14) Sugar fatty acid esters

These include those of C₁₂-C₁₈ fatty acids, e.g. sucrose monolaurate, e.g. Ryoto L-1695®, which is commercially available from e.g. Mitsubishi-Kasei Food Corp., Tokyo, Japan.

### 15) PEG sterol ethers

These include those having, e.g. from 5 to 35 [CH₂-CH₂-O] units, e.g. 20 to 30 units., e.g. Solulan® C24, which is commercially available from e.g. Amerchol.

### 16) Dioctylsodiumsulfosuccinate

This is commercially available under the trade mark Aerosol OT® from e.g. American Cyanamid Co. (Fiedler, *loc. cit*., 1, p. 118), or di-[2-ethylhexyl]-succinate (Fiedler, loc. cit., volume 1, p. 487).

### 17) Phospholipids

These include in particular lecithins (Fiedler, loc. cit., volume 2, p. 910, 1184). Suitable lecithins include, in particular, soya bean lecithins.

### 18) Salts of fatty acids, fatty acid sulfates and sulfonates

These include those of e.g. C₆-C₁₈, fatty acids, -fatty acid sulfates and sulfonates, as known and commercially available from e.g. Fluka.

### 19) Salts of acylated amino acids

These include those of C₆-C₁₈, acylated amino acids, e.g. sodium lauroyl sarcosinate, which is commercially available from e.g. Fluka.

### 20) Medium or long-chain alkyl, e.g. C₆-C₁₈, ammonium salts

These include C₆-C₁₈ acylated amino acids e.g. cetyl trimethyl ammonium bromide, which is commercially available from e.g. E. Merck AG.

The surfactant may comprise 5 to 90 % by weight of the composition of the invention; preferably 15 to 85 % by weight, more preferably 20 to 60 % by weight and even more preferably between about 35 % and about 55 % by weight.

Certain embodiments of the compositions of the invention include additives for example antioxidants, antimicrobial agents, enzyme inhibitors, stabilizers, preservatives, flavours, sweeteners and other components such as those described in Fiedler, H. P., *loc. cit*.

Preferred antioxidants include ascorbyl palmitate, butyl hydroxy anisole (BHA), butyl hydroxy toluene (BHT) and tocopherols. The antioxidant α-tocopherol (vitamin E) is especially preferred.

These additives or ingredients may comprise about 0.05 to 5% by weight of the total weight of the composition. Antioxidants, antimicrobial agents, enzyme inhibitors, stabilizers or preservatives typically provide up to about 0.05 to 1 % by weight based on the total weight of the composition. Sweetening or flavouring agents typically provide up to about 2.5 or 5% by weight based on the total weight of the composition.

The aforementioned additives can also include components that solidify a liquid microemulsion pre-concentrate (MEPC). These include solid polyethylene glycols (PEGs) and Gelucire™ products, preferably those such as like Gelucire™ 44/14 or Gelucire™ 50/13 that function as additional surfactants.

In a seventh aspect the invention provides a process for preparing a spontaneously dispersible pharmaceutical composition containing a 5-aryl-4(R)-arylcarbonylamino-pent-2-enoic acid amide substance P antagonist as an active agent, which process comprises bringing the active agent and a carrier medium comprising (1) a lipophilic component, (2) a surfactant, and optionally (3) a hydrophilic component into intimate admixture.

The carrier medium can be prepared separately before bringing the active agent into intimate admixture with the carrier medium. Alternatively the two or more of the components of the carrier medium can be mixed together with the active agent.

The spontaneously dispersible pharmaceutical composition is preferably a microemulsion preconcentrate as herein defined.

The spontaneously dispersible pharmaceutical composition preferably spontaneously or substantially spontaneously forms an o/w (oil-in-water) emulsion, e.g. microemulsion, when diluted with an aqueous medium such as water to a dilution of 1:1 to 1:300, e.g. 1:1 to 1:70, especially 1:10 to 1:70, more especially e.g. 1:10, or in the gastric juices of a patient after oral application.

In an eighth aspect the invention provides a process for the preparing a microemulsion containing a 5-aryl-4(R)-arylcarbonylamino-pent-2-enoic acid amide substance P antagonist as an active agent, which process comprises:
(i) bringing the active agent and a carrier comprising (1) a lipophilic component, (2) a surfactant, and optionally (3) a hydrophilic component into intimate admixture to form a spontaneously dispersible pharmaceutical composition; and
(ii) diluting the spontaneously dispersible pharmaceutical composition in an aqueous medium to form the microemulsion.

As mentioned above, the active agent may be present in an amount by weight of up to about 20% by weight of the composition of the invention, e.g. from about 0.05% by weight. The active agent is preferably present in an amount of 0.5 to 15 % by weight of the composition, more preferably in an amount of 1.5 to 5% by weight of the composition.

The lipophilic component preferably comprises 5 to 85 % by weight of the composition of the invention, e.g. 10 to 85%; preferably 15 to 60 % by weight, more preferably about 20 to about 40 % by weight.

The hydrophilic component (when present) may comprise 5 to 60 % by weight of the composition of the invention, e.g. 10 to 50%; preferably 10 to 40 % by weight, more preferably about 10 to about 30% by weight. It may comprise a mixture of two or more hydrophilic components. The ratio of main hydrophilic component to hydrophilic co-component is typically from about 0.5:1 to about 2: 1.

The surfactant may comprise 5 to 90% by weight of the composition of the invention; preferably 15 to 85% by weight, more preferably 20 to 60 % by weight and even more preferably between about 35% and about 55% by weight.

The relative proportion of the active agent(s), the lipophilic component(s), the surfactant(s) and the hydrophilic component(s) (when present) preferably lie within the "Microemulsion" region on a standard three way plot graph. The compositions will therefore be of high stability that are capable, on addition to an aqueous medium, of providing microemulsions, for example having a mean particle size of < 200 nm.

When the composition of the invention is a microemulsion preconcentrate it may be combined with water or an aqueous solvent medium to obtain an emulsion, for example a microemulsion. The emulsion or microemulsion may be administered enterally, for example orally, for example in the form of a drinkable solution.

When the composition of the invention is a microemulsion preconcentrate a unit dosage of the microemulsion preconcentrate is preferably used to fill orally administrable capsule shells. The capsule shells may be soft or hard capsule shells, for example made of gelatine. Each unit dosage will suitably contain from 0.1 to 100 mg active agent, for example 0.1 mg, 10 mg, 15 mg, 25 mg or 50 mg, preferably between 10 and 100 mg of the active agent, more preferably between 10 and 50 mg; for example 15, 20, 25, or 50 mg, more preferably between 5 and 20 mg, most preferably 5 or 10 mg. Such unit dosage forms are suitable for administration 1 to 5 times daily depending upon the particular purpose of therapy, the phase of therapy and the like. However, if desired, the compositions may be in drink solution form and may include water or any other aqueous system, e.g. fruit juice, milk, and the like, to provide e.g. colloidal systems, suitable for drinking, e.g. with a dilution of from about 1:10 to about 1:100.

The compositions of the invention, e.g. those in the examples hereinafter, may show good stability characteristics as indicated by standard stability trials, for example having a shelf life stability of up to one, two or three years, and even longer. One group of compositions of the invention may be of high stability that are capable, on addition to water, of providing aqueous microemulsions having an average particle size of < 200 nm (2,000 Å), e.g. <150 nm (1,500 Å), e.g. < 100 nm (1,000 Å).

The compositions of the invention exhibit especially advantageous properties when administered orally; for example in terms of consistency and high level of bioavailability obtained in standard bioavailability trials.

Pharmacokinetic parameters, for example drug substance absorption and measured for example as blood levels, also become surprisingly more predictable and problems in administration with erratic absorption may be eliminated or reduced. Additionally the pharmaceutical compositions are effective with biosurfactants or tenside materials, for example bile salts, being present in the gastro-intestinal tract. That is, the pharmaceutical compositions of the present invention are fully dispersible in aqueous systems comprising such natural tensides and thus capable of providing emulsion or microemulsion systems and/or particulate systems in situ which are stable. The function of the pharmaceutical compositions upon oral administration remain substantially independent of and/or unimpaired by the relative presence or absence of bile salts at any particular time or for any given individual. The compositions of this invention may also reduce variability in inter- and intra-patient dose response.

The utility of the compositions of the invention may be observed in standard clinical tests in, for example, known indications of active agent at dosages giving therapeutically effective active agent blood levels. Any increased bioavailability of the compositions of the invention may be observed in standard animal tests and in clinical trials.

The dose of the active agent in the compositions of the invention is of the same order as, or up to half, that used in known compositions containing the active agent. The compositions of the invention show activity at concentrations from about 0. 1 mg to about 40 mg/day of active agent, preferably from about 0.1 mg to about 20 mg/day, e.g. most preferably from about 0.1 to about 1 mg/day of active agent for respiratory disease states.

The compositions of the invention are useful for the treatment of respiratory discases, e.g. asthma and chronic bronchitis. For these indications, the appropriate dosage will, of course, vary depending upon, for example, the particular composition of the invention employed, the host, the mode of administration, and the nature and severity of the conditions being treated.

A typical dose for the active agent is from 0.1 to 1 mg/day for asthma and chronic bronchitis.

The invention is illustrated by the following Examples.

### EXAMPLES

In the following examples the pharmaceutically active agent is Compound A i.e. (4R)-4-[N'-methyl-N'-(3,5-bistrifluoro-methyl-benzoyl)amino]-4-(3,4-dichlorobenzyl)-but-2-enoic acid N-[(R)-epsilon-caprolactam-3-yl]-amide.

### Example 1

### Filled capsule (or oral solution)

The formulation contains:

| | |
|---|---|
| Compound A | 20 mg |
| Cremophor RH 40 | 215 mg |
| C8/C10 mono-/ | 130 mg |
| diglyceride | |
| Triethylcitrate | 85 mg |
| Ethanol | 50 mg |

### Example 2

### Filled capsule (or oral solution)

The formulation contains:

| | |
|---|---|
| Compound A | 10 mg |
| Cremophor RH 40 | 219 mg |
| Corn oil glycerides | 174 mg |
| Propyleneglycol | 27 mg |
| Ethanol | 75 mg |
| Tocopherol | 0.5 mg |

### Example 3

### Oral solution

| The formulation contains: | |
|---|---|
| Compound A | 10 mg/ml |
| Cremophor RH 40 | 439 mg |
| Corn oil glycerides | 343 mg |
| Propyleneglycol | 88 mg |
| Ethanol | 99 mg |
| Tocopherol | 1.0 mg |

The compositions of all three examples are prepared whereby the carrier components are mixed and the active ingredient is dissolved therein whilst stirring. The mixtures of the formulation of Examples 1 and 2 are filled into hard gelatine capsules and sealed using the Quali-Seal™ sealing technique.

Similarly, further examples are prepared as follows:

| **Excipients** | **mg/1g** |
|---|---|
| Cremophor RH40 | 423 |
| Corn oil glycerides | 253.8 |
| PEG400 | 169.2 |
| Ethanol | 94 |
| Compound A | 60 |
| | |
| Cremophor RH40 | 423 |
| Corn oil glycerides | 253.8 |
| Triethylcitrate | 169.2 |
| Ethanol | 94 |
| Compound A | 60 |
| | |
| Cremophor RH40 | 418.5 |
| C8/C10 mono-/diglycerides | 251.1 |
| PEG400 | 167.4 |
| Ethanol | 93 |
| Compound A | 70 |
| | |
| Cremophor RH40 | 400.5 |
| C8/C10 mono-/diglycerides | 240.3 |
| Propylene glycol | 160.2 |
| Ethanol | 89 |
| Compound A | 110 |
| Cremophor RH40 | 480 |
| C8/C10 mono-/diglycerides | 288 |
| Triethylcitrat | 192 |
| Compound A | 40 |
| | |
| PEG-20 glyceryl oleat | 380 |
| C8/C10 mono-/diglycerides | 285 |
| Triethylcitrate | 190 |
| Ethanol | 95 |
| Compound A | 50 |
| | |
| Cremophor RH40 | 432 |
| Propylene glycol | |
| monocaprylate | 345.6 |
| Span 80 | 86.4 |
| Triethylcitrate | 96 |
| Compound A | 40 |
| | |
| Cremophor RH40 | 405 |
| C8/C10 mono-/diglycerides | 180 |
| Propylene glycol | |
| monocaprylate | 180 |
| Ethanol | 135 |
| Compound A | 100 |
| | |
| Cremophor RH40 | 405 |
| C8/C10 mono-/diglycerides | 180 |
| Propylene glycol | |
| monocaprylate | 90 |
| Triethylcitrate | 90 |
| Ethanol | 135 |
| Compound A | 100 |
| | |
| Cremophor EL | 480 |
| Capryol 90 | 288 |
| Triethylcitrat | 192 |
| Compound A | 40 |

No phase separation or precipitation observed for any of the above compositions which are clear for at least 4 hours.

### 2. Bioavailability

Bioavailability analysis of Example 1 in a dog study shows, that single peroral doses of 20 mg Compound A given as standard capsule formulation using micronized DS (formulation A) resulted in significantly lower plasma levels of Compound A (∼50%) than given as microemulsion (formulation B). For illustration of the data see table below and figure.

Form A: Powder in Capsule, Form B: Microemulsion

| Form | tₘₐₓ[h] Median | Cₘₐₓ [ng/mL] | Cₘₐₓ/dose [(ng/mL)/(mg/kg)] | AUC(0-48h) [(ng/mL)·h] | AUC(0-48h)/dosc [(ng/mL)·h /(mg/kg)] | fᵣₑₗ (%) |
|---|---|---|---|---|---|---|
| A | 1.5 | 721±545 | 385±285 | 2622±1384 | 1397±811 | 46±27 |
| B | 1 | 2109±1136 | 1151±666 | 6779±4066 | 3683±2335 | 100±0 |

### 3. Embodiment:

In general for our microemulsions the most preferred embodiment are soft gelatine capsules. This is due to the fact that a number of microemulsion excipients e.g. propylene glycol, are incompatible with hard gelatin capsules making them brittle. Soft gelatine capsules contain a large amount of softener and therefore compatibility is better.

## Claims

1. A spontaneously dispersible pharmaceutical composition comprising a 5-aryl-4(R)-arylcarbonylamino-pent-2-enoic acid amide substance P antagonist and a carrier medium comprising a lipophilic component and a surfactant.

2. A spontaneously dispersible pharmaceutical composition as claimed in claim 1, wherein the carrier medium further comprises a hydrophilic component.

3. A pharmaceutical composition as claimed in claim 1 or 2 where the 5-aryl-4(R)-arylcarbonylamino-pent-2-enoic acid amide substance P antagonist is (4R)-4-[N'-methyl-N'-(3,5-bistrifluoro-methyl-benzoyl)amino]-4-(3,4-dichlorobenzyl)-but-2-enoic acid N-[(R)-epsilon-caprolactam-3-yl]-amide.

4. A pharmaceutical composition comprising (4R)-4-[N'-methyl-N'-(3,5-bistrifluoromethyl-benzoyl)aminol-4-(3,4-dichlorobenzyl)-but-2-enoic acid N-[(R)-epsilon-caprolactam-3-yl]-amide as active agent and a carrier medium comprising a lipophilic component, a surfactant and optionally a hydrophilic component, said composition being in a form that is suitable for oral administration.

5. A composition as claimed in claim 1 or 4 wherein the lipophilic component comprises C₈-C₁₀ fatty acid monoglycerides and diglycerides or a refined glycerol-transesterified corn oil.

6. A composition as claimed in any one of claims 2, 4 and 5 wherein the hydrophilic component comprises propylene glycol.

7. A composition as claimed in any one of claims 1, 4, 5 and 6 wherein the surfactant comprises a polyethyleneglycol-hydrogenated castor oil.

8. A spontaneously dispersible pharmaceutical composition comprising about 0.05 to about 20 % by weight of (4R)-4-[N'-methyl-N'-(3,5-bistrifluoro-methyl-benzoyl)amino]-4-(3,4-dichlorobenzyl)-but-2-enoic acid N-[(R)-epsilon-caprolactam-3-yl]-amide, about 5 to about 85 % by weight of a lipophilic component, about 5 to about 90 % by weight of a surfactant, and optionally about 5 to about 60 % by weight of a hydrophilic component, all weights based on the total composition.

9. A composition as claimed in any one of claims 1 to 8 in the form of a microemulsion preconcentrate.

10. A composition as claimed in any one of claims 1 to 8 in the form of a microemulsion.

11. A composition according to any preceding claim in unit dosage form.

12. A composition according to claim 11 in soft or hard gelatin encapsulated form.

13. A process for preparing a spontaneously dispersible pharmaceutical composition containing a 5-aryl-4(R)-arylcarbonylamino-pent-2-enoic acid amide substance P antagonist as an active agent, which process comprises bringing the active agent and a carrier medium comprising (1) a lipophilic component, (2) a surfactant, and optionally (3) a hydrophilic component into intimate admixture.

14. A process for the preparing a microemulsion containing a 5-aryl-4(R)-arylcarbonylamino-pent-2-enoic acid amide substance P antagonist as an active agent, which process comprises:
(i) bringing the active agent and a carrier comprising (1) a lipophilic component, (2) a surfactant, and optionally (3) a hydrophilic component into intimate admixture to form a spontaneously dispersible pharmaceutical composition; and
(ii) diluting the spontaneously dispersible pharmaceutical composition in an aqueous medium to form the microemulsion.

## Patentansprüche

1. Spontan dispergierbare pharmazeutische Zusammensetzung, die einen 5-Aryl-4(R)-aryl-carbonylamino-pent-2-ensäureamid-Substanz P-Antagonisten und ein Trägermedium umfasst, das einen lipophilen Bestandteil und ein grenzflächenaktives Mittel umfasst.

2. Spontan dispergierbare pharmazeutische Zusammensetzung nach Anspruch 1, wobei das Trägermedium weiterhin einen hydrophilen Bestandteil umfasst.

3. Pharmazeutische Zusammensetzung nach Anspruch 1 oder 2, wobei der 5-Aryl-4(R)-aryl-carbonylamino-pent-2-ensäureamid-Substanz P-Antagonist (4R)-4-[N'-Methyl-N'-(3,5-bistrifluor-methyl-benzoyl)amino]-4-(3,4-dichlorbenzyl)-but-2-ensäure-N-[(R)-epsilon-caprolactam-3-yl]-amid ist.

4. Pharmazeutische Zusammensetzung, die (4R)-4-[N'-Methyl-N'-(3,5-bistrifluormethyl-benzoyl)amino]-4-(3,4-dichlorbenzyl)-but-2-ensäure-N-[(R)-epsilon-caprolactam-3-yl]-amid als Wirkstoff und ein Trägermedium umfasst, das einen lipophilen Bestandteil, ein grenzflächenaktives Mittel und wahlweise einen hydrophilen Bestandteil umfasst, wobei die Zusammensetzung in einer Form vorliegt, die zur oralen Verabreichung geeignet ist.

5. Zusammensetzung nach Anspruch 1 oder 4, wobei der lipophile Bestandteil C₈-C₁₀-Fettsäuremonoglyzeride und -diglyzeride oder ein raffiniertes Glyzerintransverestertes Maiskeimöl umfasst.

6. Zusammensetzung nach einem der Ansprüche 2, 4 und 5, wobei der hydrophile Bestandteil Propylenglykol umfasst.

7. Zusammensetzung nach einem der Ansprüche 1, 4, 5 und 6, wobei das grenzflächenaktive Mittel ein Polyethylenglykol-hydriertes Rizinusöl umfasst.

8. Spontan dispergierbare pharmazeutische Zusammensetzung, die ungefähr 0,05 bis ungefähr 20 Gew.-% (4R)-4-[N'-Methyl-N'-(3,5-bistrifluor-methyl-benzoyl)amino]-4-(3,4-dichlorbenzyl)-but-2-ensäure-N-[(R)-epsilon-caprolactam-3-yl]-amid, ungefähr 5 bis ungefähr 85 Gew.-% eines lipophilen Bestandteils, ungefähr 5 bis ungefähr 90 Gew.-% eines grenzflächenaktiven Mittels und wahlweise ungefähr 5 bis ungefähr 60 Gew.-% eines hydrophilen Bestandteils umfasst, wobei sich alle Gewichtsangaben auf die Gesamtzusammensetzung beziehen.

9. Zusammensetzung nach einem der Ansprüche 1 bis 8 in Form eines Mikroemulsionsvorkonzentrats.

10. Zusammensetzung nach einem der Ansprüche 1 bis 8 in Form einer Mikroemulsion.

11. Zusammensetzung nach einem der vorangehenden Ansprüche in Form einer Dosiereinheit.

12. Zusammensetzung nach Anspruch 11 in einer in Weich- oder Hartgelatine eingekapselten Form.

13. Verfahren zum Herstellen einer spontan dispergierbaren pharmazeutischen Zusammensetzung, die einen 5-Aryl-4(R)-aryl-carbonylamino-pent-2-ensäureamid-Substanz P-Antagonisten als Wirkstoff umfasst, wobei das Verfahren umfasst, den Wirkstoff und ein Trägermedium, das (1) einen lipophilen Bestandteil, (2) ein grenzflächenaktives Mittel und wahlweise (3) einen hydrophilen Bestandteil umfasst, in enge Zumischung zu bringen.

14. Verfahren zum Herstellen einer Mikroemulsion, die einen 5-Aryl-4(R)-aryl-carbonylamino-pent-2-ensäureamid-Substanz P-Antagonisten als Wirkstoff enthält, wobei das Verfahren Folgendes umfasst:
(i) Bringen des Wirkstoffs und eines Trägers, der (1) einen lipophilen Bestandteil, (2) ein grenzflächenaktives Mittel und wahlweise (3) einen hydrophilen Bestandteil umfasst, in enge Zumischung, um eine spontan dispergierbare pharmazeutische Zusammensetzung zu bilden; und
(ii) Verdünnen der spontan dispergierbaren pharmazeutischen Zusammensetzung in einem wässrigen Medium, um die Mikroemulsion zu bilden.

## Revendications

1. Composition pharmaceutique spontanément dispersible comprenant un antagoniste de substance P amide d'acide 5-aryl-4(R)-aryl-carbonylamino-pent-2-énoïque et un milieu de support comportant un composant lipophile et un tensioactif.

2. Composition pharmaceutique spontanément dispersible selon la revendication 1, dans laquelle le milieu de support comprend en outre un composant hydrophile.

3. Composition pharmaceutique selon la revendication 1 ou 2, dans laquelle l'antagoniste de substance P amide d'acide 5-aryl-4(R)-aryl-carbonylamino-pent-2-énoïque est le N-[(R)-epsilon-caprolactam-3-yl]-amide d'acide (4R)-4-[N'-méthyl-N'-(3,5-bistrifluoro-méthyl-benzoyl)amino]-4-(3,4-dichlorobenzyl)-but-2-énoïque.

4. Composition pharmaceutique comprenant du N-[(R)-epsilon-caprolactam-3-yl]-amide d'acide (4R)-4-[N'-méthyl-N'-(3,5-bistrifluoro-méthyl-benzoyl)amino]-4-(3,4-dichlorobenzyl)-but-2-énoïque en tant qu'agent actif et un milieu de support comportant un composant lipophile, un tensioactif et en option un composant hydrophile, ladite composition se présentant sous une forme qui est appropriée pour une administration par voie orale.

5. Composition selon la revendication 1 ou 4, dans laquelle le composant lipophile comprend des monoglycérides et des diglycérides d'acide gras en C₈-C₁₀ ou une huile de maïs raffinée transestérifiée avec un glycérol.

6. Composition selon l'une quelconque des revendications 2, 4 et 5, dans laquelle le composant hydrophile comprend du propylèneglycol.

7. Composition selon l'une quelconque des revendications 1, 4, 5 et 6, dans laquelle le tensioactif comprend une huile de ricin hydrogénée avec du polyéthylèneglycol.

8. Composition pharmaceutique spontanément dispersible comprenant environ 0,05 à environ 20 % en poids de N-[(R)-epsilon-caprolactam-3-yl]-amide d'acide (4R)-4-[N'-méthyl-N'-(3,5-bistrifluoro-méthyl-benzoyl)amino]-4-(3,4-dichlorobenzyl)-but-2-énoïque, environ 5 à environ 85 % en poids d'un composant lipophile, environ 5 à environ 90 % en poids d'un tensioactif, et en option environ 5 à environ 60 % en poids d'un composant hydrophile, tous les poids étant ramenés à la composition totale.

9. Composition selon l'une quelconque des revendications 1 à 8 sous la forme d'un préconcentré de microémulsion.

10. Composition selon l'une quelconque des revendications 1 à 8 sous la forme d'une microémulsion.

11. Composition selon l'une quelconque des revendications précédentes sous une forme monodose.

12. Composition selon la revendication 11 sous une forme encapsulée dans de la gélatine molle ou dure.

13. Procédé de préparation d'une composition pharmaceutique spontanément dispersible comprenant un antagoniste de substance P amide d'acide 5-aryl-4(R)-aryl-carbonylamino-pent-2-énoïque en tant qu'agent actif, ledit procédé comprenant la mise en mélange intime de l'agent actif et d'un milieu de support comportant (1) un composant lipophile, (2) un tensioactif et en option (3) un composant hydrophile.

14. Procédé de préparation d'une microémulsion contenant un antagoniste de substance P amide d'acide 5-aryl-4(R)-aryl-carbonylamino-pent-2-énoïque en tant qu'agent actif, ledit procédé comprenant les étapes consistant à :
(i) mettre en mélange intime l'agent actif et un support comportant (1) un composant lipophile, (2) un tensioactif et en option (3) un composant hydrophile pour former une composition pharmaceutique spontanément dispersible ; et
(ii) diluer la composition pharmaceutique spontanément dispersible dans un milieu aqueux pour former la microémulsion.
